(19) Europäisches Patentamt
European Patent Office
Office européen des brevets

(11) EP 2 028 176 B1

(12) **EUROPÄISCHE PATENTSCHRIFT**

(45) Veröffentlichungstag und Bekanntmachung des Hinweises auf die Patenterteilung:
**09.03.2011 Patentblatt 2011/10**

(51) Int Cl.:
***C07C 209/84*** *(2006.01)* ***C07C 211/46*** *(2006.01)*

(21) Anmeldenummer: **08013972.8**

(22) Anmeldetag: **05.08.2008**

(54) **Verfahren zur Reinigung von aromatischen Aminen**

Method for purifying aromatic amines

Procédé destiné au nettoyage d’amines aromatiques

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **18.08.2007 DE 102007039091**

(43) Veröffentlichungstag der Anmeldung:
**25.02.2009 Patentblatt 2009/09**

(73) Patentinhaber: **Bayer MaterialScience AG**
**51368 Leverkusen (DE)**

(72) Erfinder:
- **Sommer, Knut, Dr.**
**47804 Krefeld (DE)**
- **Gehlen, Franz-Ulrich**
**47839 Krefeld (DE)**
- **Lehner, Peter, Dr.**
**45481 Mülheim/Ruhr (DE)**
- **Düx, Andre**
**50321 Brühl (DE)**
- **Marotz, Benie**
**40227 Düsseldorf (DE)**

(56) Entgegenhaltungen:
**EP-A- 1 845 079** **JP-A- 2005 350 388**
**US-A1- 2005 080 294**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 028 176 B1

## Beschreibung

Die Erfindung betrifft ein Verfahren zur Reinigung aromatischer Amine, die durch Reduktion von aromatischen Nitroverbindungen erhalten werden. Unter aromatischen Aminen werden hier solche Verbindungen verstanden, die mindestens eine Aminogruppe an einem aromatischen Ring tragen. Letzterer kann beliebig substituiert bzw. mit anderen aromatischen Ringen kondensiert sein.

Die Reinigung dieser aromatischen Amine erfolgt in der vorliegenden Erfindung in der Weise, dass das aus der Reduktion erhaltene Produktgemisch zunächst durch eine dem Fachmann bekannte Phasentrennung von der Hauptmenge des während der Reduktion entstandenen Reaktionswassers befreit und das so erhaltene organische Verfahrensprodukt, im Folgenden als rohes Amin oder Roh-Amin bezeichnet, danach zusammen mit einer wässrigen Lösung eines Alkalihydroxids in eine Destillationskolonne geleitet wird, die wie folgt arbeitet: Leichtsieder werden als Kopfprodukt, Hochsieder als Sumpfprodukt und das gereinigte aromatische Amin, im Folgenden als reines Amin oder Rein-Amin bezeichnet, in einem Seitenstrom entnommen. Die Kolonne besteht aus mindestens drei Sektionen, nämlich einem oberen Verstärkungsteil, einem unteren Verstärkungsteil sowie einem Abtriebsteil. Der Ablauf aus dem Abtriebsteil wird mit Hilfe eines Hauptverdampfers teilweise verdampft. Ein Teilstrom der nicht verdampften Flüssigkeit wird dann in einem Nachverdampfer eingeengt, wobei die Gasphase wieder in die Kolonne zurückgeführt wird und die Flüssigphase teilweise oder vollständig ausgeschleust wird. Auf diese Weise wird mit minimalem apparativem und energetischem Aufwand eine maximale Abreicherung des wertvollen Amins im Sumpf der Kolonne gewährleistet.

Aromatische Amine sind wichtige Zwischenprodukte, welche preiswert und in großen Mengen zur Verfügung stehen müssen. Daher müssen beispielsweise für die Herstellung von Anilin Anlagen mit sehr großen Kapazitäten gebaut werden. Anilin ist beispielsweise ein wichtiges Zwischenprodukt bei der Herstellung von Methylendiphenyldiisocyanat (MDI) und wird im großtechnischen Maßstab in der Regel durch katalytische Reduktion von Nitrobenzol mit Wasserstoff hergestellt, wie es in DE-A 2201528, DE-A 3414714, US 3136818, EP-B1 0 696 573, EP-B1 0 696 574 beschrieben ist.

Bei katalytischen Reduktionen zur Herstellung aromatischer Amine werden neben den Zielprodukten auch Wasser und organische Nebenkomponenten gebildet. Diese organischen Nebenkomponenten müssen vor einer weiteren Verwendung der aromatischen Amine abgetrennt werden. Sie umfassen zum einen die Gruppe der "Leichtsieder", d. h. Verbindungen bzw. azeotrop siedende Gemische von Einzelkomponenten mit Siedepunkten unter denen des herzustellenden Amins, und zum anderen die Gruppe der "Hochsieder", d. h. Verbindungen bzw. azeotrop siedende Gemische von Einzelkomponenten mit Siedepunkten über denen des herzustellenden Amins. Im Fall der Reduktion von Nitrobenzol zu Anilin (Sdp. = 184 °C) sind Beispiele für die Gruppe der Leichtsieder das Benzol (Sdp. = 80 °C) und für die Gruppe der Hochsieder das Diphenylamin (Sdp. = 302 °C). Diese beiden als Beispiele genannten Verunreinigungen lassen sich durch Destillation einfach abtrennen, weil ihre Siedepunkte von denen des herzustellenden

Amins sehr verschieden sind ($\Delta T_S$ = 104 K bzw. 118 K). Wesentlich problematischer ist die Abtrennung von solchen Nebenkomponenten, deren Siedepunkte denen des herzustellenden Amins sehr ähnlich sind, weil hier der destillative Aufwand erheblich größer wird. Im Fall der Reduktion von Nitrobenzol stellt insbesondere die Trennung von Phenol (Sdp. = 182 °C) und dem Zielprodukt Anilin (Sdp. =184 °C) eine große Herausforderung für die Destillationstechnik dar, was sich in der Verwendung langer Destillationskolonnen mit großer Trennstufenzahl und hohen Rücklaufverhältnissen mit entsprechend hohem Investitions- und Energieaufwand widerspiegelt. Verbindungen mit phenolischen Hydroxygruppen, d. h. Verbindungen, die mindestens eine Hydroxygruppe an einem aromatischen Ring tragen, können generell bei der Aufarbeitung aromatischer Amine problematisch sein. Im Fall des Anilins sind hier neben dem bereits erwähnten Phenol insbesondere die verschiedenen Aminophenole zu nennen.

Die Reinigung aromatischer Amine ist daher nicht trivial und besitzt große industrielle Bedeutung. Neuere Ansätze widmen sich insbesondere der erwähnten Problematik im Zusammenhang mit Verbindungen mit phenolischen Hydroxygruppen. Der Lösungsansatz besteht darin, die Verbindungen mit phenolischen Hydroxygruppen durch Reaktion mit geeigneten Basen in die entsprechenden Salze zu überführen, welche sich als nichtflüchtige Verbindungen wesentlich leichter abtrennen lassen. Zu diesem Zweck erfolgt ein Einsatz von Alkalihydroxiden zur Extraktion oder ein Alkalihydroxidzusatz bei der Destillation. So wird in JP-A-49-035341 ein Verfahren beschrieben, bei dem das zu reinigende Amin, dort Anilin, mit festen Alkalihydroxiden in einem Festbett in Kontakt gebracht und erst anschließend in die Destillation geleitet wird, bzw. bei dem die Destillation in Gegenwart des festen Alkalihydroxides in Anteilen von 0,1 - 3 Massenprozent, bezogen auf die zu destillierende Anilinmenge, durchgeführt wird. Hierdurch wird die Abtrennung kritischer Komponenten wie der Aminophenole vereinfacht. Nachteilig bei diesem Verfahren ist jedoch der Einsatz von hohen molaren Überschüssen der festen Alkalihydroxide in Bezug auf die zu entfernenden sauren Nebenkomponenten bzw. die Unmöglichkeit der genauen Dosierung der alkalischen Verbindungen. Dies kann einerseits bei Überdosierung zu Korrosionsproblemen, Ausfällungen und hochviskosen Sumpfphasen in der Destillationskolonne und andererseits bei Unterdosierung zu einer unvollständigen Entfernung der kritischen Komponenten führen.

JP-A-08-295654 beschreibt als Alternative zur destillativen Entfernung von Verbindungen mit phenolischen Hydroxygruppen aus Anilin eine Extraktion mit verdünnter wässriger Natronlauge oder Kalilauge (Konzentration 0,1 - 0,7 Massenprozent, bezogen auf die Masse der Alkalihydroxid-Lösung), wodurch das Phenol als Alkaliphenolat

größtenteils in die wässrige Phase transferiert wird, die durch die anschließende Phasentrennung abgetrennt wird. Für eine effektive Reduzierung des Phenolgehaltes ist ein molares Verhältnis NaOH: Phenol im Bereich von 3 : 1 - 100 : 1 erforderlich. Nachteilig bei diesem Verfahren sind der hohe NaOH-Verbrauch (molare Überschüsse), das Anfallen - infolge der geringen Konzentration der Alkalihydroxid-Lösungen - sehr großer Mengen alkaliphenolathaltigen Abwassers, was zu zusätzlichen Entsorgungskosten führt, sowie ein zusätzlicher Investitionsaufwand für die Extraktion.

US-A-2005 080294 beschreibt ein Verfahren zur Abtrennung von Verbindungen mit phenolischen Hydroxygruppen (dort "phenolic compounds" genannt) von aromatischen Aminen, bei dem dem zu reinigenden Amin vor der Destillation eine Base im molaren Verhältnis von 1 : 1 bis zu 4 : 1 bezogen auf die "phenolic compounds" zugesetzt wird, optional in Gegenwart von Polyolen. US-A-2005 080294 lehrt nicht im Einzelnen, was mit den Salzen, die bei Reaktion der "phenolic compounds" mit den Basen entstehen, geschieht. In Beispiel 6 wird lediglich erwähnt, dass überschüssiges festes KOH durch Zusatz von Polyethylenglykol (PEG) in Lösung gebracht wird. Welche Konsequenzen damit verbunden sind, ist US-A-2005 080294 nicht zu entnehmen. Auf die Salze der "phenolic compounds" selbst geht US-A-2005 080294 gar nicht ein.

Salze jedoch, und zwar nicht nur überschüssige Base, sondern auch die Salze der Verbindungen mit phenolischen Hydroxygruppen, sind im Allgemeinen in aromatischen Aminen nur wenig löslich, sodass die große Gefahr besteht, dass sie sich in der Destillationskolonne, im Sumpf der Destillationskolonne und/oder im Verdampfer der Destillation über die Löslichkeitsgrenze hinweg anreichern und dann ausfallen. Solche Feststoffniederschläge können den Destillationsprozess empfindlich stören, sodass eine Unterbrechung des Destillationsvorgangs erforderlich wird, was in der großtechnischen Produktion, die kontinuierlich betrieben wird, zu erheblichen Schwierigkeiten und sogar zu Produktionsausfällen führen kann. US-A-2005 080294 befasst sich mit dem Problem der Zuverlässigkeit und Standzeit des Verfahrens jedoch nicht. Der Fachmann erfährt aus US-A-2005 080294 auch nicht, dass die Anwesenheit der während der Reaktion der Verbindungen mit phenolischen Hydroxygruppen mit den Basen entstehenden Salze zu Feststoffabscheidung, Fouling und/oder starkem Viskositätsanstieg während der Destillation führen kann. Auf Details der Destillationstechnik geht US-A-2005 080294 gar nicht ein. Der Fachmann erfährt aus US-A-2005 080294 daher nicht, wie er diese mit hoher Wahrscheinlichkeit auftretenden Probleme lösen soll. US-A-2005 080294 lehrt nur die optionale zusätzliche Zugabe von PEG, um überschüssiges festes KOH in Lösung zu bringen. Eine solche Zugabe von PEG in die Destillation ist aufgrund der hohen Kapazitäten bei der Herstellung aromatischer Amine (insbesondere Anilin) jedoch wirtschaftlich nicht tragbar. Der Einsatz des in US-A 2005 080294 beschriebenen Verfahrens in einem kontinuierlichem Produktionsprozess wird nicht beschrieben.

JP-A-2005 350388 befasst sich ganz allgemein mit der Verbesserung der Anilinaufarbeitung. Es wird ein Verfahren beschrieben, bei dem ein Teil des Sumpfes der Anilin-Destillationskolonne aus dieser abgeführt und separat, d. h. in einem vom eigentlichen Verdampfer der Kolonne verschiedenen zweiten Verdampfer, in die Gasphase überführt wird. Die so erhaltene Gasphase wird in die Reinanilinkolonne zurückgeführt; nicht verdampfbare Hochsiederanteile werden abgetrennt. Auf diese Weise können die Temperatur der Anilin-Destillationskolonne relativ gering gehalten und Verschmutzungen verringert werden; der Anilinverlust bei der Hochsiederabtrennung wird ebenfalls verringert. Nachteilig bei diesem Verfahren ist, dass Leichtsieder und Wasser vor der eigentlichen Anilin-Destillationskolonne in einem apparativ aufwändigen Verfahren in einer Entwässerungskolonne separat durch eine zusätzliche Destillation abgetrennt werden müssen. JP-A-2005 350388 erwähnt nicht die besondere Problematik mit Verbindungen mit phenolischen Hydroxygruppen für die Destillation. Der Patentanmeldung ist daher auch nicht zu entnehmen, ob deren Abtrennung vom Zielprodukt Anilin mit dem dort beschriebenen Verfahren verbessert werden kann.

EP-A-07075103 beschreibt ein Verfahren zur Reinigung von Anilin durch Zugabe einer wässrigen Alkalimetallhydroxid-Lösung vor oder während der Destillation. EP-A-07075104 beschreibt ein Verfahren zur Reinigung von Anilin durch Extraktion mit wässriger Alkalimetallhydroxid-Lösung. Im Unterschied zur vorliegenden Anmeldung beschreibt keine der beiden genannten die nachfolgend erläuterte Fahrweise der teilweisen bis vollständigen Ausschleusung des Sumpfes der Anilin-Destillationskolonne und teilweisen Verdampfung desselben über zwei seriell oder parallel geschaltete Verdampfer ($E^1$) und ($E^2$). Durch diese im Folgenden noch näher beschriebene Vorgehensweise wird mit minimalem apparativem und energetischem Aufwand eine maximale Abreicherung des wertvollen Amins im Sumpf der Destillationskolonne erreicht.

Die Aufgabe der vorliegenden Erfindung war daher, ein Verfahren zur Reinigung von aromatischen Aminen bereitzustellen, das die Abtrennung der Verbindungen mit phenolischen Hydroxygruppen verbessert, ohne dass dabei Probleme wie Feststoffabscheidung, Fouling und/oder starker Viskositätsanstieg während der Destillation auftreten, die Produktausbeute und die Standzeiten verbessert und gleichzeitig einen geringen apparativen Aufwand mit geringem Energieverbrauch gewährleistet.

Diese Aufgabe wurde gelöst durch ein Verfahren zur Reinigung aromatischer Amine, die durch Reduktion von aromatischen Nitroverbindungen hergestellt werden, wobei das aus der Reduktion erhaltene Produktgemisch folgenden Reinigungsschritten in der nachfolgenden Reihenfolge unterzogen wird:

I. Abtrennung des während der Reduktion entstandenen Reaktionswassers durch Phasentrennung, wobei sich im

so gewonnenen rohen Amin ein der Löslichkeit von Wasser im rohen Amin entsprechender Restgehalt an Wasser einstellt, der von Druck und Temperatur sowie der genauen Zusammensetzung des rohen Amins abhängig ist und der zwischen 2 und 10 Massenprozent, bevorzugt zwischen 4 und 10 Massenprozent, bezogen auf die Masse des rohen Amins, liegt,

II. Vermischen des aus Schritt I erhaltenen rohen Amins mit einer wässrigen Lösung eines Alkalihydroxids,

III. Zufuhr der Mischung aus Schritt II in eine Destillationskolonne (K), wobei diese Destillationskolonne (K) mindestens ein oberes Verstärkungsteil ($VT^2$), ein unteres Verstärkungsteil ($VT^1$) sowie ein Abtriebsteil (AT) enthält,

IV. teilweise bis vollständige Entnahme des Sumpfproduktes aus dem Bereich unterhalb des Abtriebsteils (AT),

V. teilweise Verdampfung dieses aus Schritt IV entnommenen Sumpfproduktes in einem Hauptverdampfer ($E^1$) und wenigstens einem nachgeschalteten Nachverdampfer ($E^2$), wobei der Nachverdampfer ($E^2$) bei einer höheren Temperatur betrieben wird als der Hauptverdampfer ($E^1$), bis zu einem Restgehalt an aromatischen Amin im Ablauf des Nachverdampfers ($E^2$) von 0 bis 98 Massenprozent,

VI. vollständige Rückleitung der in Schritt V erhaltenen Gasphasen und teilweise Rückführung der Flüssigphasen in die Kolonne (K), entweder unter- oder oberhalb des Abtriebsteils (AT) und teilweise oder vollständige Ausschleusung der im Nachverdampfer ($E^2$) anfallenden Flüssigphase.

Vorteilhaft ist das erfindungsgemäße Verfahren besonders dann wenn Amine der Formel

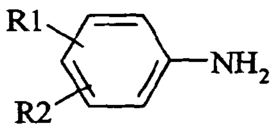

gereinigt werden sollen, in welcher R1 und R2 unabhängig voneinander Wasserstoff, Methyl oder Ethyl bedeuten, wobei R1 zusätzlich Amino bedeuten kann. Ganz besonders vorteilhaft ist die Erfindung, wenn durch Reduktion von Nitrobenzol erhaltenes Anilin gereinigt werden soll.

Vorteilhaft ist das erfindungsgemäße Verfahren besonders dann, wenn die Alkalihydroxide als wässrige Lösungen eingesetzt werden und der Gehalt an Alkalihydroxid in diesen Lösungen > 0,7 Massenprozent und ≤ 55 Massenprozent ist.

Vorteilhaft ist das erfindungsgemäße Verfahren besonders dann, wenn die Absolutmenge an zugesetzten Alkalihydroxiden in einem Bereich von

$$0{,}01 < n(MOH) / [\sum\{i \cdot n(Ar(OH)_i)\}] < 1{,}5$$

liegt, wobei

n = Stoffmenge,
M = Alkalimetall,
$Ar(OH)_i$ = Verbindung mit i phenolischen Hydroxygruppen und
i eine natürliche Zahl bedeuten.

Der Term

$$[\sum\{i \cdot n(Ar(OH)_i)\}]$$

bezeichnet demnach die Summe aller phenolischen Hydroxygruppen im rohen Amin, beispielsweise die Summe der Hydroxygruppen aus Phenol und den verschiedenen Aminophenolen. Zur Bestimmung dieses Terms ist eine Bestimmung der Konzentration der Verbindungen mit phenolischen Hydroxygruppen im rohen Amin vor der Vermischung mit der wässrigen Alkalihydroxid-Lösung erforderlich. Dies geschieht durch gängige analytische Methoden, bevorzugt durch Gaschromatographie.

Vorteilhaft ist das erfindungsgemäße Verfahren besonders dann, wenn die Destillationskolonne (K) bei einem absoluten Druck von 10 bis 1000 mbar und mit einem Rücklaufverhältnis von 0,05 bis 3 betrieben wird.

Vorteilhaft ist das erfindungsgemäße Verfahren besonders dann, wenn der Nachverdampfer ($E^2$) beim gleichen Druck wie der Hauptverdampfer ($E^1$) betrieben wird.

Vorteilhaft ist das erfindungsgemäße Verfahren besonders dann, wenn der Nachverdampfer ($E^2$) geeignet ist für die Verarbeitung von Belag-bildenden Produkten, d. h. solchen Produkten, die zur Bildung von Ablagerungen auf Oberflächen neigen. Mögliche Ausführungen sind z. B. Platten- oder Rohrbündelwärmeaustauscher mit Zwangsumlauf, Dünnschicht-, Fallfilm- oder Wendelrohrverdampfer.

Vorteilhaft ist das erfindungsgemäße Verfahren besonders dann, wenn der Nachverdampfer ($E^2$) in regelmäßigen Abständen mit Wasser, bevorzugt Reaktionswasser, gespült wird. Wie häufig solche Reinigungen erforderlich sind, hängt vom verwendeten Verdampfertyp ab.

Vorteilhaft ist das erfindungsgemäße Verfahren besonders dann, wenn der Sumpfablauf des Hauptverdampfers ($E^1$) mit Wasser, bevorzugt Reaktionswasser, extrahiert wird.

Vorteilhaft ist das erfindungsgemäße Verfahren besonders dann, wenn sehr niedrige Wassergehalte (z. B. c ($H_2O$) << 1000 ppm) im Rein-Amin angestrebt werden, weil dieses Ziel durch Ausführung der Destillationskolonne (K) als Trennwandkolonne auf sehr einfache Weise erreicht werden kann.

Das generelle Funktionsprinzip von Trennwandkolonnen wird beispielsweise in G. Kaibel, "Distillation Columns with Vertical Partitions", Chem. Eng. Technol. 1987, 10, 92 - 98 und G. Kaibel, "Industrieller Einsatz von Trennwandkolonnen und thermisch gekoppelten Destillationskolonnen", Chemie Ingenieur Technik 2003, 75, 1165 - 1166 beschrieben.

Nachfolgend wird das erfindungsgemäße Verfahren beschrieben:

Die genannten aromatischen Amine werden in dem erfindungsgemäßen Verfahren durch katalytische Reduktion der entsprechenden Nitroverbindungen hergestellt. Letztere sind diejenigen Verbindungen, bei denen der aromatische Ring anstelle der Aminogruppe(n) der Zielverbindung eine Nitrogruppe bzw. mehrere Nitrogruppen trägt.

In dem erfindungsgemäßen Verfahren kann die Reduktion der Nitroverbindungen zu den gewünschten aromatischen Aminen durch sämtliche technisch realisierbaren Methoden erfolgen, bevorzugt durch Reduktion mit Wasserstoff (Hydrierung), besonders bevorzugt durch Gasphasen-Hydrierung.

Ganz besonders bevorzugt wird die Gasphasen-Hydrierung an ortsfesten, heterogenen Trägerkatalysatoren, wie z. B. Pd auf Aluminiumoxid oder Kohlenstoffträgern, in Festbettreaktoren bei einem absoluten Druck von 1 - 50 bar und einer Temperatur im Bereich von 150 - 600 °C unter adiabaten Bedingungen in Kreisgasfahrweise, d. h. unter Rückführung von während der Hydrierung nicht umgesetzten Wasserstoffs, durchgeführt. Ein solches Verfahren wird beispielsweise in EP-B1 0 696 573 und EP-B1 0 696 574 beschrieben.

Das während der Reduktion der aromatischen Nitroverbindung zum Amin entstandene Reaktionswasser wird zunächst durch eine dem Fachmann bekannte Phasentrennung abgetrennt. Hierbei wird der Wassergehalt des rohen Amins auf Werte abgereichert, die der Löslichkeit von Wasser im rohen Amin unter den gegebenen physikalischen Randbedingungen (Druck, Temperatur, Zusammensetzung) entsprechen; dieser Restgehalt an Reaktionswasser liegt zwischen 2 und 10 Massenprozent, bevorzugt zwischen 4 und 10 Massenprozent. Eine Entfernung von anderen Leichtsiedern erfolgt in dieser Stufe nicht.

Die sich an die Phasentrennung anschließenden Reinigungsschritte werden im Folgenden mit Hilfe der Zeichnungen im Anhang detailliert erläutert. Es zeigen:

Abb. 1: eine schematische Darstellung der Destillationskolonne mit zweistufiger Energiezufuhr im Kolonnensumpf

Abb. 2. eine schematische Darstellung der Destillationskolonne mit zweistufiger Energiezufuhr im Kolonnensumpf und $H_2O$-Zugabe in der zweiten Verdampferstufe

Abb. 3: eine schematische Darstellung der Destillationskolonne mit zweistufiger Energiezufuhr im Kolonnensumpf und Zwischenextraktion

Abb. 4: eine schematische Darstellung der Trennwandkolonne mit zweistufiger Energiezufuhr im Kolonnensumpf

Abb. 5: eine schematische Darstellung der Trennwandkolonne mit zweistufiger Energiezufuhr im Kolonnensumpf und Zwischenextraktion

Die Abbildungen zeigen lediglich mögliche Ausführungsformen des erfindungsgemäßen Verfahrens und sind nicht als einschränkend zu verstehen.

Abb. 1 zeigt, wie das so erhaltene rohe Amin (1) mit einer wässrigen Alkalihydroxid-Lösung (2) - zur weitgehenden Überführung von Verbindungen mit phenolischen Hydroxygruppen in die entsprechenden Alkalisalze - in einen Zulauferhitzer ($E^3$) vermischt und als Zulauf (3) direkt in eine Destillationskolonne (K) geleitet wird. Als Alkalihydroxid-Lösungen (2) können bevorzugt Natronlauge oder Kalilauge, besonders bevorzugt Natronlauge eingesetzt werden. Grundsätzlich können aber wässrige Lösungen aller Alkalimetalle eingesetzt werden. Prinzipiell könnten an Stelle der Alkalimetallhydroxide auch andere wasserlösliche basische Verbindungen wie z. B. Erdalkalimetallhydroxide, Alkali- oder Erdalkalimetallcarbonate oder -hydrogencarbonate eingesetzt werden. Unter wirtschaftlichen Gesichtspunkten sind Alkalimetallhydroxide jedoch vorzuziehen.

Der Gehalt an Alkalihydroxid in diesen Lösungen ist > 0,7 Massenprozent und ≤ 55 Massenprozent, bevorzugt ≥ 1 Massenprozent und ≤ 50 Massenprozent, besonders bevorzugt ≥ 5 Massenprozent und ≤ 35 Massenprozent. Die absolute zuzugebende Menge an Alkalihydroxid wird dabei so gewählt, dass es in einem Bereich von

$$0{,}01 < n(MOH) / [\sum\{i \cdot n(Ar(OH)_i)\}] < 1{,}5$$

bevorzugt von

$$0{,}50 < n(MOH) / [\sum\{i \cdot n(Ar(OH)_i)\}] < 1{,}0$$

besonders bevorzugt von

$$0{,}90 < n(MOH) / [\sum\{i \cdot n(Ar(OH)_i)\}] < 1{,}0$$

vorliegt. Durch die Verwendung maximal geringer Überschüsse an Alkalihydroxid (in bevorzugten Ausführungsformen des erfindungsgemäßen Verfahrens werden Überschüsse an Alkalihydroxid sogar vollständig vermieden) wird die Bildung von Feststoffabscheidungen in der Destillationskolonne, im Sumpf der Destillationskolonne und/oder im Verdampfer der Destillation, welche den Destillationsprozess empfindlich stören oder sogar ganz unmöglich machen können, verhindert.

Die Destillationskolonne (K) wird bei einem absoluten Druck von 10 bis 1000 mbar, bevorzugt von 50 bis 800 mbar, besonders bevorzugt von 100 bis 500 mbar und mit einem Rücklaufverhältnis von 0,05 bis 3, bevorzugt von 0,1 bis 2, besonders bevorzugt von 0,1 bis 0,9 betrieben. Mit Rücklaufverhältnis wird in diesem speziellen Fall das Verhältnis der Flüssigkeitsmenge, die am oberen Ende des unteren Verstärkungsteils (Segment $VT^1$) aufgegeben wird, zur entnommenen Flüssigkeitsmenge im Seitenstrom (4) bezeichnet. Die Destillationskolonne (K) enthält mindestens ein unteres Verstärkungsteil ($VT^1$), ein oberes Verstärkungsteil ($VT^2$) sowie ein Abtriebsteil (AT). Im unteren Verstärkungsteil ($VT^1$) erfolgt die Abtrennung der Hochsieder. Diese gelangen zusammen mit einem Teil des Amins in den Abtriebsteil (AT), wo sie aufkonzentriert werden. Leichtsieder und Wasser werden im oberen Verstärkungsteil ($VT^2$) aufkonzentriert, die Brüden (12) werden über Kopf entnommen und zur Trennung von Leichtsiedern und Wasser durch n Kondensatoren ($C^1$), ($C^2$), ... ($C^n$) geleitet (dass in der Abbildung nur zwei Kondensatoren gezeigt sind, die über Leitung (14) verbunden sind, ist nicht als Einschränkung zu verstehen), wobei n eine natürliche Zahl ist und im Bereich von 1 bis 5, bevorzugt bis 3, besonders bevorzugt bis 2 liegen kann. Die aus den n Kondensatoren anfallenden n Kondensate (hier Strom 13 und 15) werden zusammengeführt und einer Phasentrennung unterworfen. Hierbei wird die wässrige Phase (7) ausgetragen und entweder an eine andere Stelle im Prozess zurückgeführt, bevorzugt in die in Schritt I. beschriebene Phasentrennung, oder entsorgt. Die organische, im Wesentlichen aus dem aromatischen Amin bestehende Phase (16) wird in den Kopf der Destillationskolonne (K) zurückgeführt. In einer besonderen Ausführungsform wird ein Teil der n Kondensate (20 und 21) zwecks zusätzlicher Leichtsieder-Abtrennung ausgeschleust. In einer weiteren Ausführungsform, die dem Fachmann als Dephlegmator-Fahrweise bekannt ist, werden Leichtsieder mit dem Restbrüdenstrom (8) teilweise bis vollständig (0,01 - 100 %) ausgeschleust. Das Rein-Amin wird in einem Seitenstrom (4) zwischen den beiden Verstärkungsteilen ($VT^1$) und ($VT^2$) entnommen.

Der Ablauf aus dem Abtriebsteil (AT) wird mit Hilfe des Hauptverdampfers ($E^1$) teilweise verdampft. Ein Teilstrom oder der Gesamtstrom der nicht verdampften Flüssigkeit (11) wird dann in einem Nachverdampfer ($E^2$) eingeengt, wobei die Gasphase (10) wieder in die Kolonne zurückgeführt wird und die Flüssigphase (5) ausgeschleust wird. Dabei wird

der Nachverdampfer ($E^2$) bei einer um bis zu 100 K höheren Temperatur betrieben als der Hauptverdampfer ($E^1$). Auf diese Weise wird der Sumpf der Destillationskolonne (K) effektiv und schonend eingeengt. Die gebildeten Alkalisalze der Verbindungen mit phenolischen Hydroxygruppen reichern sich im Sumpf der Destillationskolonne (K) bzw. des Hauptverdampfers ($E^1$) an. Diese Anreicherung erfolgt bevorzugt bis knapp unterhalb der Löslichkeitsgrenze, welche von den physikalischen Randbedingungen (Druck, Temperatur, Zusammensetzung) abhängt. (Gegebenenfalls noch vorhandene geringe Restmengen an nicht abreagierten, d. h. ihre OH-Funktion(en) noch besitzenden, Verbindungen mit phenolischen Hydroxygruppen werden, abhängig vom jeweiligen Siedepunkt, zusammen mit den Hoch- oder Leichtsiedern abgetrennt.) Die zweite Verdampferstufe wird bei dem gleichen Druck wie die erste betrieben. Die Hochsieder im Ablauf der ersten Verdampferstufe (11) werden aber noch weiter aufkonzentriert, d. h. der Nachverdampfer ($E^2$) wird bevorzugt bei höherer Temperatur betrieben als der Hauptverdampfer ($E^1$). Obwohl die Einengung des Sumpfes des Nachverdampfers ($E^2$) bis über die Löslichkeitsgrenze der Salze der Verbindungen mit phenolischen Hydroxygruppen hinweg betrieben werden kann, sind an dieser Stelle keine Probleme durch evtl. auftretende Feststoffabscheidungen zu befürchten, weil der Nachverdampfer ($E^2$) in bevorzugten Ausführungsformen des erfmdungsgemäßen Verfahrens für die Verarbeitung von Belag-bildenden Produkten geeignet ist, besonders leicht zu reinigen ist und in regelmäßigen Abständen mit Wasser, bevorzugt Reaktionswasser, gespült wird. Die Ausschleusung aus dem Nachverdampfer ($E^2$) erfolgt durch dem Fachmann bekannte Austragsvorrichtungen wie Pumpen oder Schnecken. Mögliche Ausführungsformen für den Nachverdampfer ($E^2$) sind z. B. dem Fachmann bekannte Platten- oder Rohrbündelwärmeaustauscher mit Zwangsumlauf, Dünnschicht-, Fallfilm- oder Wendelrohrverdampfer. Durch diese Vorgehensweise wird bei minimalem apparativem und energetischem Aufwand eine maximale Abreicherung des wertvollen Amins im Sumpf der Destillationskolonne erzielt. Die Aminrestgehalte im Ablauf des Nachverdampfers ($E^2$) werden auf Werte von 0 bis 98 Massenprozent, bevorzugt 15 bis 80 Massenprozent, besonders bevorzugt 20 bis 60 Massenprozent und ganz besonders bevorzugt 20 bis 40 Massenprozent eingestellt.

Die Gasphase (9), die aus dem Hauptverdampfer ($E^1$) erhalten wird, wird unterhalb des Abtriebteils (AT) wieder in die Destillationskolonne (K) zurückgeführt. Die Gasphase (10), die aus dem Nachverdampfer ($E^2$) erhalten wird, wird ebenfalls unterhalb des Abtriebsteils (AT) der Destillationskolonne (K) wieder in die Destillationskolonne (K) zurückgeleitet, und der Sumpfablauf (5) des Nachverdampfers ($E^2$) wird entsorgt.

Abb. 2 zeigt eine alternative Ausgestaltung der in Abb. 1 beschriebenen Destillationskolonne, wobei über einen Zustrom (6) Wasser in den Nachverdampfer ($E^2$) eingeführt wird, um eventuell vorhandene Ablagerungen zu entfernen. In diesem Fall wird die Spüllösung gemeinsam mit dem übrigen Rückstand (5) entsorgt.

Abb. 3 ist eine alternative Ausführung der Abb. 2, in der der Sumpfablauf (11) mit Wasser (6), bevorzugt Reaktionswasser, extrahiert wird. Dabei werden die Alkalisalze aus der organischen Phase herausgewaschen und in die wässrige Phase überführt. Dazu wird das Gemisch (17) nach Durchlaufen eines Kühlers ($E^4$) in einen Extraktor (Ex) geleitet, wo organische und wässrige Phase anschließend mittels einer dem Fachmann bekannten Phasentrennung getrennt werden. Die so erhaltene organische Phase (18) wird in den Nachverdampfer ($E^2$) geleitet, und die wässrige Phase (19) wird mit dem übrigen Abwasser entsorgt. Auf diese Weise wird ein störungsfreier kontinuierlicher Betrieb der Anlage gewährleistet und die Bildung von Niederschlägen, z. B. von Alkaliphenolaten, welche den Destillationsprozess empfindlich stören oder sogar ganz unmöglich machen können, vermieden.

Werden an den Wassergehalt des gereinigten Amins besondere Anforderungen gestellt (z. B. $c(H_2O) << 1000$ ppm), so ist eine Ausführung der Destillationskolonne als Trennwandkolonne bevorzugt. Abb. 4 ist daher eine alternative Ausführung zu der Destillationskolonne (K) aus Abb. 1, wobei jedoch eine Trennwandkolonne eingesetzt wird. Die Trennwandkolonne enthält, wie in der Verfahrensvariante nach Abb. 1 auch, einen oberen Verstärkungsteil ($VT^2$) sowie ein Abtriebsteil (AT) und anstelle des unteren Verstärkungsteil ($VT^1$) mindestens vier Sektionen im Bereich der Trennwand, sodass die Gesamtzahl an Sektionen in dieser Ausführungsform mindestens sechs beträgt. Im Bereich der Trennwand dient die Sektion ($VT^1LO$) zur Abreicherung hoch siedender Verbindungen, die im Zulaufstrom (3) enthalten sind, die Sektion ($VT^1LU$) zur Abreicherung von Leichtsiedern, die im Zulaufstrom (3) enthalten sind, insbesondere Wasser, die Sektion ($VT^1RO$) zur weiteren Abreicherung von Leichtsiedern, die in der aus ($VT^2$) ablaufenden Flüssigkeit enthalten sind, damit diese nicht ins Amin gelangen, und die Sektion ($VT^1RU$) zur Abreicherung von Hochsiedern, die in den aus (AT) kommenden Dämpfen enthalten sind. Der Energieeintrag erfolgt analog zu der Verfahrensvariante entsprechend Abb. 1.

Eine Alternative der Verfahrensweise nach Abb. 4 stellt die Abb. 5 dar. In Abb. 5 ist ebenfalls wie in Abb. 3 eine Vorrichtung (Ex) zur Extraktion des Sumpfablaufs mit Wasser, bevorzugt Reaktionswasser, vorgesehen. Die Trennwandkolonne enthält, wie in der Verfahrensvariante nach Abb. 3 auch, einen oberen Verstärkungsteil ($VT^2$) sowie ein Abtriebsteil (AT). Im Unterschied zum in Abb. 3 skizzierten Verfahren befinden sich anstelle des unteren Verstärkungsteil ($VT^1$) im Bereich einer Trennwand mindestens fünf Sektionen, sodass die Gesamtzahl an Sektionen in dieser Ausführungsform mindestens sieben beträgt. Im Bereich der Trennwand dient die Sektion ($VT^1LO$) zur Abreicherung hoch siedender Verbindungen, die im Zulaufstrom (3) enthalten sind, die Sektionen ($VT^1LU^1$) und ($VT^1LU^2$) dienen zur Abreicherung von Leichtsiedern, die im Zulaufstrom (3) oder im Dampfstrom (10) enthalten sind, insbesondere Wasser, die Sektion ($VT^1RO$) dient zur weiteren Abreicherung von Leichtsiedern, die in der aus ($VT^2$) ablaufenden Flüssigkeit

enthalten sind, damit diese nicht ins Amin gelangen, und einer Sektion ($VT^1RU$) zur Abreicherung von Hochsiedern, die in den aus (AT) kommenden Dämpfen enthalten sind. Der Energieeintrag erfolgt analog zu der Verfahrensvariante entsprechend Abb. 3. Allerdings werden die Brüden (10) aus dem Nachverdampfer ($E^2$) aufgrund Ihres Wassergehaltes zwischen den Sektionen ($VT^1LU^1$) und ($VT^1LU^2$) der Trennwandkolonne zugeführt. Hierdurch wird verhindert, dass das in den Brüden (10) des Nachverdampfers ($E^2$) enthaltene Wasser in das Amin gelangt.

Das vorstehend beschriebene Verfahren beinhaltet eine praktikable Lösung des Trennproblems im Zusammenhang mit Verbindungen mit phenolischen Hydroxygruppen und gewährleistet bei minimalem apparativem und energetischem Aufwand eine maximale Abreicherung des wertvollen Amins im Sumpf der Destillationskolonne.

## Beispiele

### Beispiel 1 (erfindungsgemäß): Destillation bei 360 mbar und R/E = 0,3 (aus Simulationsrechnung)

Ein Rohanilinstrom (1,994 kg/h) bestehend aus

| Komponente | Anteil in Massenprozent |
|---|---|
| Anilin | 97,600 |
| Wasser | 2,000 |
| Benzol | 0,065 |
| Phenol | 0,077 |
| Diphenylamin | 0,139 |
| Sonstige Nebenkomponenten | 0,119 |

wird mit einer 10%igen Natronlauge (6,1 g/h) vermischt. Das so erhaltene Gemisch wird einer Destillationskolonne (Durchmesser: 70 mm) bestehend aus

1. einem oberen Verstärkungsteil mit 10 theoretischen Stufen
2. einem unteren Verstärkungsteil mit 12 theoretischen Stufen
3. einem Abtriebsteil mit 12 theoretischen Stufen

zugeführt. Die Kolonne wird bei einem Druck von 360 mbar betrieben. Das Kondensationssystem der Kolonne ist zweistufig, wobei die erste Stufe bei einer Kondensationstemperatur von 65 °C, die zweite bei einer Temperatur von 45 °C betrieben wird. Das anfallende Kondensat wird auf 30 °C gekühlt und einer Phasentrennung zugeführt. Die organische (gleichzeitig auch die schwere) Phase wird wieder am Kopf der Kolonne aufgegeben.

Unterhalb des oberen Verstärkungsteils wird Anilin (1,95 kg/h) mit einer Reinheit von 99,94 % und folgenden Verunreinigungen erhalten:

| Komponente | Anteil in Massenprozent |
|---|---|
| Wasser | 0,0371 |
| Benzol | 0,0018 |
| Phenol | 0,0040 |
| Sonstige Nebenkomponenten | 0,0171 |

Das Verhältnis des entnommenen Produktstromes zum Flüssigkeitsstrom, der aus dem oberen Verstärkungsteil ablief, betrug 0,23, woraus sich ein Rücklaufverhältnis von 0,3 ergab.

Der Energieeintrag in die Kolonne erfolgte zweistufig. In einer ersten Stufe wurde die aus der Kolonne ablaufende Flüssigkeit in einem Fallfilmverdampfer (Hauptverdampfer) bis an die Löslichkeitsgrenze des Natriumphenolats (3 Massenprozent) eingeengt. Dabei wurde die Anilinkonzentration auf 92 Massenprozent abgereichert. Die Sumpftemperatur betrug 152 °C. Anschließend wurde das Sumpfprodukt zur weiteren Aufkonzentrierung und Minimierung der Anilinverluste einem Dünnschichtverdampfer (Nachverdampfer) zugeführt. Dabei wurde der Anilingehalt auf 49,3 Massenprozent abgereichert, wodurch sich die Natriumphenolat-Konzentration auf 19,5 Massenprozent erhöhte. Die Sumpftemperatur stieg auf 165 °C an.

**Beispiel 2 (erfindungsgemäß): Destillation bei 150 mbar und R/E = 0,8 (aus Simulationsrechnung)**

Bei sonst gleichen Bedingungen wie unter Beispiel 1 wird die Kolonne bei einem Kopfdruck von 150 mbar betrieben. Das Kondensationssystem der Kolonne ist zweistufig, wobei die erste Stufe bei einer Kondensationstemperatur von 45 °C, die zweite bei einer Temperatur von 35 °C betrieben wird. Das anfallende Kondensat wird auf 30 °C gekühlt und einer Phasentrennung zugeführt. Die organische (gleichzeitig die schwere) Phase wird wieder am Kopf der Kolonne aufgegeben.

Unterhalb des oberen Verstärkungsteils wird Anilin (1,95 kg/h) mit einer Reinheit von 99,96 % und folgenden Verunreinigungen erhalten:

| Komponente | Anteil in Massenprozent |
|---|---|
| Wasser | 0,0200 |
| Benzol | 0,0009 |
| Phenol | 0,0017 |
| Sonstige Nebenkomponenten | 0,0174 |

Das Verhältnis des entnommenen Produktstromes zum Flüssigkeitsstrom, der aus dem oberen Verstärkungsteil ablief, betrug 0,44, was einem Rücklaufverhältnis von 0,8 entspricht.

Der Energieeintrag in die Kolonne erfolgte zweistufig. In einer ersten Stufe wurde die aus der Kolonne ablaufende Flüssigkeit in einem Fallfilmverdampfer (Hauptverdampfer) bis an die Löslichkeitsgrenze des Natriumphenolats (3 Massenprozent) eingeengt. Dabei wurde die Anilinkonzentration auf 92 Massenprozent abgereichert. Die Sumpftemperatur betrug 129 °C. Anschließend wurde das Sumpfprodukt zur weiteren Aufkonzentrierung und Minimierung der Anilinverluste einem Dünnschichtverdampfer (Nachverdampfer) zugeführt. Dabei wurde der Anilingehalt auf 49,3 Massenprozent abgereichert wodurch sich die Natriumphenolat-Konzentration auf 19,5 Massenprozent erhöhte. Die Sumpftemperatur stieg auf 141 °C an.

**Beispiel 3 (erfindungsgemäß): Destillation im Labor bei 400 mbar mit einem Dünnschichtverdampfer als Nachverdampfer**

Ein Zulaufgemisch (Zusammensetzung siehe Tabelle unten), das die Zusammensetzung des Ablaufs des Hauptverdampfers ($E^1$) repräsentiert, wurde mittels einer einstufigen Verdampfung bei einem Absolutdruck von 400 mbar und einer Kopftemperatur von 150 °C in einem Dünnschichtverdampfer, der hier als Nachverdampfer ($E^2$) dient, thermisch getrennt. Hierzu wurde kontinuierlich ein Zulaufstrom von 800 g/h über eine Dosierpumpe in den Dünnschichtverdampfer eingespeist. Die Beheizung des Verdampfers (ca. 160 °C) wurde so eingestellt, dass 86 - 87 Massenprozent des Zulaufstroms als Destillat anfielen. Das Destillat wurde nach einer einstufigen Kondensation komplett entnommen. Der verbleibende, flüssige Massenstrom (13 - 14 Massenprozent) wurde am Sumpf des Dünnschichtverdampfers entnommen.

Ergebnisse der Laborversuche zur Aufarbeitung des Zulaufgemisches im Dünnschichtverdampfer:

| Komponente | Anteil in Massenprozent | | |
|---|---|---|---|
| | im Zulauf | im Destillat | im Sumpf |
| Anilin | 94,428 | 99,600 | 60,000 |
| Phenol | 0,044 | 0,000 | 0,600 |
| Diphenylamin | 2,528 | 0,400 | 17,000 |
| Natriumphenolat | 3,000 | 0,000 | 22,400 |

## Patentansprüche

1. Verfahren zur Reinigung aromatischer Amine, die durch Reduktion von aromatischen Nitroverbindungen hergestellt werden, wobei das aus der Reduktion erhaltene Produktgemisch folgenden Reinigungsschritten in der nachfolgenden Reihenfolge unterzogen wird:

   I. Abtrennung des während der Reduktion entstandenen Reaktionswassers durch Phasentrennung, wobei sich im so gewonnenen rohen Amin ein der Löslichkeit von Wasser im rohen Amin entsprechender Restgehalt an

Wasser einstellt, der von Druck und Temperatur sowie der genauen Zusammensetzung des rohen Amins abhängig ist und der zwischen 2 und 10 Massenprozent, bezogen auf die Masse des rohen Amins, liegt,
II. Vermischen des aus Schritt I erhaltenen rohen Amins mit einer wässrigen Lösung eines Alkalihydroxids,
III. Zufuhr der Mischung aus Schritt II in eine Destillationskolonne (K), wobei diese Destillationskolonne (K) mindestens ein oberes Verstärkungsteil ($VT^2$), ein unteres Verstärkungsteil ($VT^1$) sowie ein Abtriebsteil (AT) enthält,
IV. teilweise bis vollständige Entnahme des Sumpfproduktes aus dem Bereich unterhalb des Abtriebsteils (AT),
V. teilweise Verdampfung dieses aus Schritt IV entnommenen Sumpfproduktes in einem Hauptverdampfer ($E^1$) und wenigstens einem nachgeschalteten Nachverdampfer ($E^2$), wobei der Nachverdampfer ($E^2$) bei einer höheren Temperatur betrieben wird als der Hauptverdampfer ($E^1$), bis zu einem Restgehalt an aromatischen Amin im Ablauf des Nachverdampfers ($E^2$) von 0 bis 98 Massenprozent,
VI. vollständige Rückleitung der in Schritt V erhaltenen Gasphasen und teilweise Rückführung der Flüssigphasen in die Kolonne (K), entweder unter- oder oberhalb des Abtriebsteils (AT) und teilweise oder vollständige Ausschleusung der im Nachverdampfer ($E^2$) anfallenden Flüssigphase.

**2.** Verfahren nach Anspruch 1, wobei das aromatische Amin Anilin und die aromatische Nitroverbindung Nitrobenzol ist.

**3.** Verfahren nach einem der Ansprüche 1 oder 2, wobei die Alkalihydroxide als wässrige Lösungen eingesetzt werden und der Gehalt an Alkalihydroxid in diesen Lösungen > 0,7 Massenprozent und ≤ 55 Massenprozent ist.

**4.** Verfahren nach einem der Ansprüche 1 bis 3, wobei die Absolutmenge an zuzugebendem Alkalihydroxid so gewählt wird, dass es in einem Bereich von

$$0{,}01 < n(MOH) / [\sum\{i \cdot n(Ar(OH)_i)\}] < 1{,}5$$

vorliegt, wobei

n für Stoffmenge,
M für Alkalimetall,
Ar(OH); für Verbindung mit i phenolischen Hydroxygruppen und
i für eine natürliche Zahl steht,

und wobei zur Bestimmung der Konzentration der Verbindungen mit phenolischen Hydroxygruppen das Verfahrensprodukt aus Verfahrensschritt I vor der Vermischung mit der wässrigen Alkalihydroxid-Lösung in regelmäßigen Abständen analysiert wird.

**5.** Verfahren nach einem der Ansprüche 1 bis 4, wobei die Destillationskolonne (K) bei einem absoluten Druck von 10 bis 1000 mbar und mit einem Rücklaufverhältnis von 0,05 bis 3 betrieben wird.

**6.** Verfahren nach einem der Ansprüche 1 bis 5, wobei der Nachverdampfer ($E^2$) bei gleichem Druck wie der Hauptverdampfer ($E^1$) betrieben wird.

**7.** Verfahren nach einem der Ansprüche 1 bis 6, wobei der Nachverdampfer ($E^2$) geeignet ist für die Verarbeitung von Belag-bildenden Produkten.

**8.** Verfahren nach einem der Ansprüche 1 bis 7, wobei der Nachverdampfer ($E^2$) in regelmäßigen Abständen mit Wasser gespült wird.

**9.** Verfahren nach einem der Ansprüche 1 bis 8, wobei der Sumpfablauf des Hauptverdampfers ($E^1$) mit Wasser extrahiert und die organische gewaschene Phase in den Nachverdampfer ($E^2$) geleitet wird.

**10.** Verfahren nach einem der Ansprüche 1 bis 9, wobei die Destillationskolonne (K) als Trennwandkolonne ausgeführt wird.

**Claims**

1. Process for the purification of aromatic amines prepared by reduction of aromatic nitro compounds, the product mixture obtained from the reduction being subjected to the following purification steps in the order below:

   I. separating off the water of reaction formed during reduction, by phase separation, where the residual water content established in the resultant crude amine is that corresponding to the solubility of water in the crude amine, and is dependent on pressure and temperature and also on the precise composition of the crude amine, and is between 2 and 10 per cent by mass, based on the mass of the crude amine,
   II. mixing the crude amine obtained from step I with an aqueous solution of an alkali metal hydroxide,
   III. feeding the mixture from step II to a distillation column (K), this distillation column (K) comprising at least one upper rectifying section ($VT^2$),a lower rectifying section ($VT^1$) and a stripping section (AT),
   IV. removing some or all of the bottom product from the region below the stripping section (AT),
   V. partially evaporating this bottom product taken from step IV in a main evaporator ($E^1$) and at least one downstream re-evaporator ($E^2$), the re-evaporator ($E^2$) being operated at a higher temperature than the main evaporator ($E^1$), down to a residual aromatic amine content in the outflow from the re-evaporator ($E^2$) of 0 to 98 per cent by mass,
   VI. completely recycling the gas phases obtained in step V, and partially recycling the liquid phases, to the column (K), either below or above the stripping section (AT), and partially or completely removing the liquid phase obtained in the re-evaporator ($E^2$).

2. Process according to Claim 1, where the aromatic amine is aniline and the aromatic nitro compound is nitrobenzene.

3. Process according to either of Claims 1 and 2, where the alkali metal hydroxides are used in the form of aqueous solutions and the alkali metal hydroxide content of these solutions is > 0.7 per cent by mass and ≤ 55 per cent by mass.

4. Process according to any of Claims 1 to 3, where the absolute amount of alkali metal hydroxide to be added is selected such that it is in a range of

$$0.01 < n(MOH)/[\sum\{i \cdot n(Ar(OH)_i)\}] < 1.5,$$

   where

   n is molar amount of substance,
   M is alkali metal,
   $Ar(OH)_i$ is compound having i phenolic hydroxyl groups and
   i is a natural number,

   and where the concentration of the compounds having phenolic hydroxyl groups is determined by analysing at regular intervals the process product from process step I prior to mixing with the aqueous alkali metal hydroxide solution.

5. Process according to any of Claims 1 to 4, where the distillation column (K) is operated at an absolute pressure of 10 to 1000 mbar and with a reflux ratio of 0.05 to 3.

6. Process according to any of Claims 1 to 5, where the re-evaporator ($E^2$ is operated at the same pressure as the main evaporator ($E^1$).

7. Process according to any of Claims 1 to 6, where the re-evaporator ($E^2$) is suitable for the processing of deposit-forming products.

8. Process according to any of Claims 1 to 7, where the re-evaporator ($E^2$) is flushed with water at regular intervals.

9. Process according to any of Claims 1 to 8, wherein the bottom outflow from the main evaporator ($E^1$) is extracted with water and the organic washed phase is passed into the re-evaporator ($E^2$).

**10.** Process according to any of Claims 1 to 9, where the distillation column (K) is configured as a dividing-wall column.

**Revendications**

**1.** Procédé de purification d'amines aromatiques, qui sont fabriquées par réduction de composés nitro aromatiques, dans lequel le mélange de produits obtenu à partir de la réduction est soumis aux étapes de purification suivantes dans l'ordre suivant :

I. la séparation de l'eau de réaction formée pendant la réduction par séparation de phases, une teneur résiduelle en eau qui correspond à la solubilité de l'eau dans l'amine brute s'ajustant dans l'amine brute ainsi obtenue, qui dépend de la pression et de la température, ainsi que de la composition exacte de l'amine brute, et qui est comprise entre 2 et 10 pourcent en masse, par rapport à la masse de l'amine brute,
II. le mélange de l'amine brute obtenue à l'étape I avec une solution aqueuse d'un hydroxyde alcalin,
III. l'introduction du mélange de l'étape II dans une colonne de distillation (K), cette colonne de distillation (K) contenant au moins une zone d'enrichissement supérieure ($VT^2$), une zone d'enrichissement inférieure ($VT^1$) et une zone de rectification (AT),
IV. le soutirage partiel à total du produit de fond de la zone sous la zone de rectification (AT),
V. l'évaporation partielle de ce produit de fond soutiré de l'étape IV dans un évaporateur principal ($E^1$) et au moins un évaporateur secondaire en aval ($E^2$) l'évaporateur secondaire ($E^2$) étant exploité à une température plus élevée que l'évaporateur principal ($E^1$) , jusqu'à une teneur résiduelle en amine aromatique dans la sortie de l'évaporateur secondaire ($E^2$) de 0 à 98 pourcent en masse,
VI. le recyclage total des phases gazeuses obtenues à l'étape V et le recyclage partiel des phases liquides dans la colonne (K), en dessous ou au-dessus de la zone de rectification (AT), et l'évacuation partielle ou totale de la phase liquide formée dans l'évaporateur secondaire ($E^2$).

**2.** Procédé selon la revendication 1, dans lequel l'amine aromatique est l'aniline et le composé nitro aromatique le nitrobenzène.

**3.** Procédé selon l'une quelconque des revendications 1 ou 2, dans lequel les hydroxydes alcalins sont utilisés sous la forme de solutions aqueuses et la teneur en hydroxyde alcalin dans ces solutions est > 0,7 pourcent en masse et ≤ 55 pourcent en masse.

**4.** Procédé selon l'une quelconque des revendications 1 à 3, dans lequel la quantité absolue d'hydroxyde alcalin à ajouter est choisie de manière à se situer dans une plage de

$$0{,}01 < n(MOH)/[\sum\{i \cdot n(Ar(OH)_i)\}] < 1{,}5$$

n représentant une quantité de matière,
M représentant un métal alcalin,
$Ar(OH)_i$ représentant un composé qui contient i groupes hydroxy phénoliques et
i représentant un nombre naturel,

et dans lequel le produit du procédé de l'étape de procédé I est analysé à intervalles réguliers avant le mélange avec la solution aqueuse d'hydroxyde alcalin pour déterminer la concentration des composés contenant des groupes hydroxy phénoliques.

**5.** Procédé selon l'une quelconque des revendications 1 à 4, dans lequel la colonne de distillation (K) est exploitée à une pression absolue de 10 à 1 000 mbar et avec un taux de reflux de 0,05 à 3.

**6.** Procédé selon l'une quelconque des revendications 1 à 5, dans lequel l'évaporateur secondaire ($E^2$ est exploité à la même pression que l'évaporateur principal ($E^1$).

**7.** Procédé selon l'une quelconque des revendications 1 à 6, dans lequel l'évaporateur secondaire ($E^2$) est approprié pour le traitement de produits formant un dépôt.

**8.** Procédé selon l'une quelconque des revendications 1 à 7, dans lequel l'évaporateur secondaire ($E^2$) est rincé à intervalles réguliers avec de l'eau.

**9.** Procédé selon l'une quelconque des revendications 1 à 8, dans lequel la sortie de fond de l'évaporateur principal ($E^1$) est extraite avec de l'eau et la phase organique lavée est conduite dans l'évaporateur secondaire ($E^2$).

**10.** Procédé selon l'une quelconque des revendications 1 à 9, dans lequel la colonne de distillation (K) est conçue sous la forme d'une colonne à paroi de séparation.

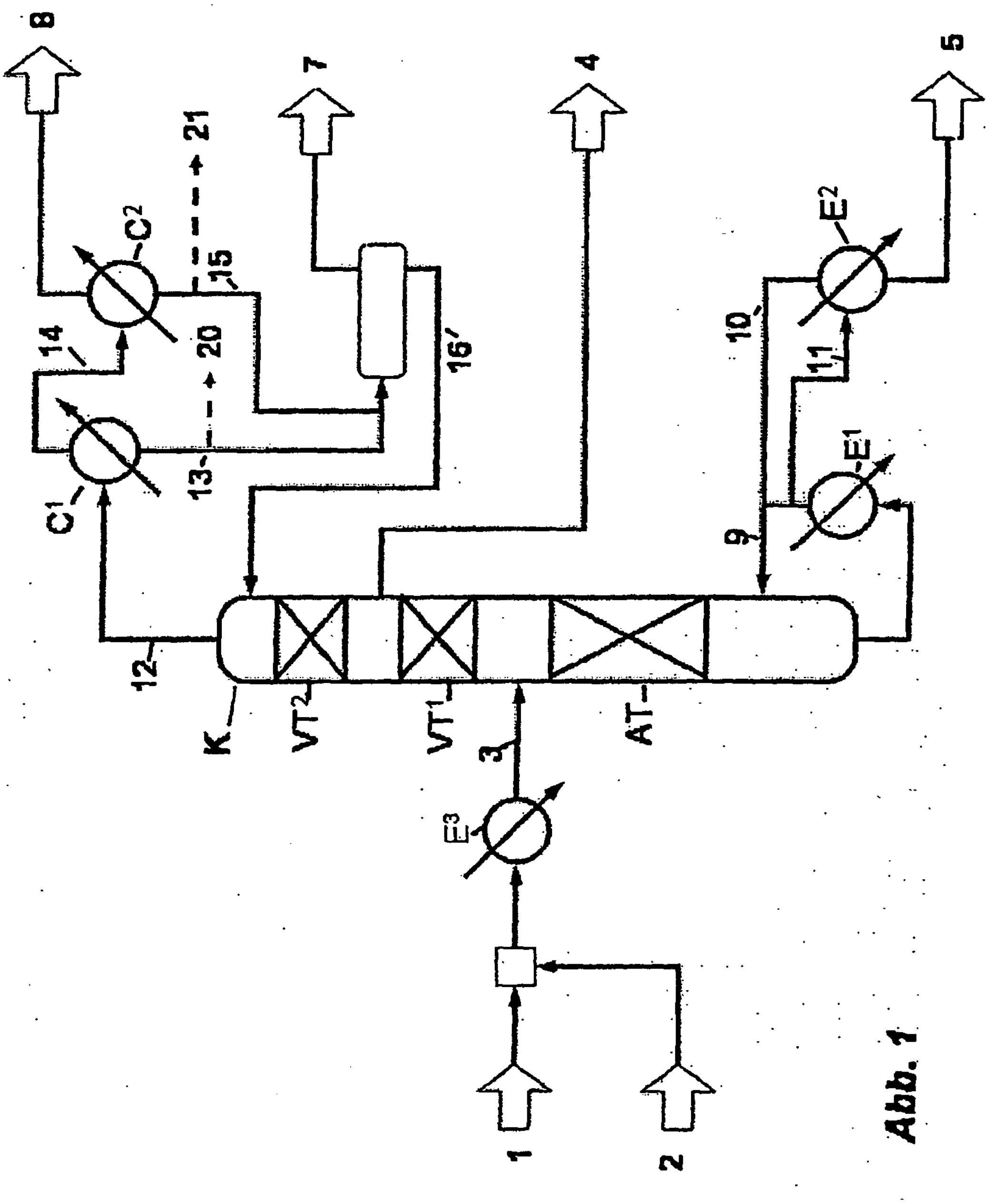


Abb. 1

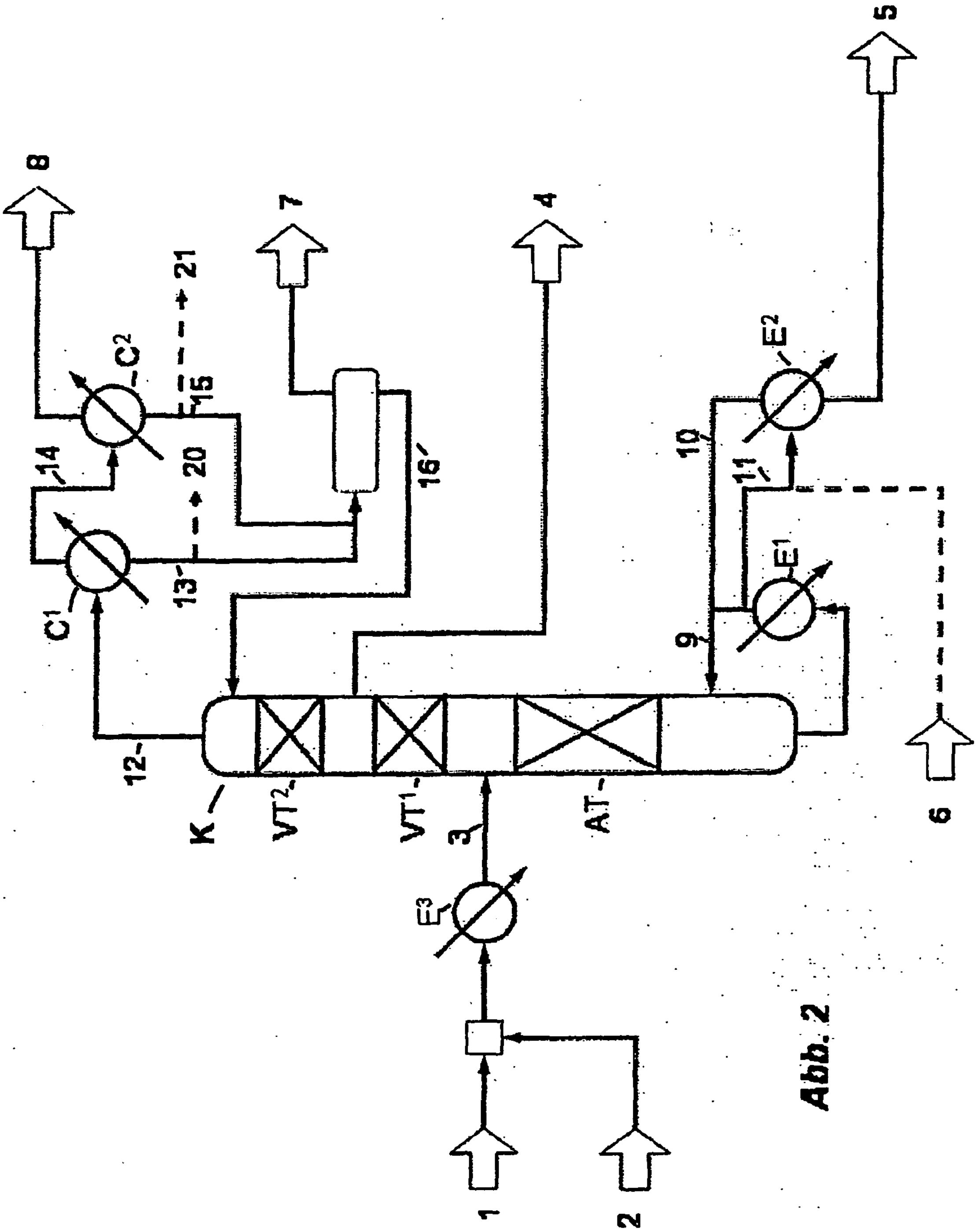


Abb. 2

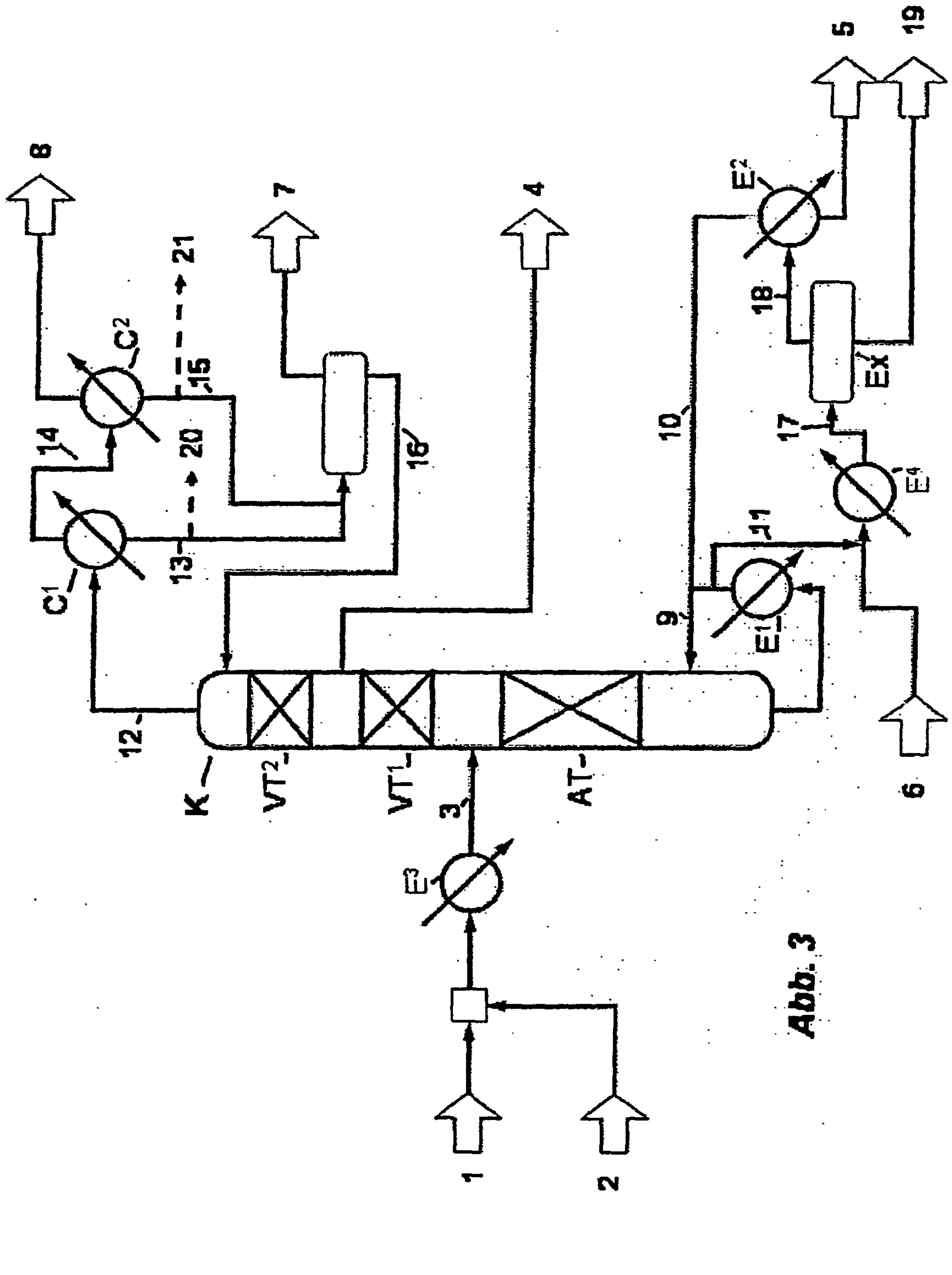
1
2
3
4
5
6
7
8
9
10
11
12
13
14
15
16
17
18
19
20
21
K
VT2
VT1
AT
C1
C2
E1
E2
E3
E4
EX

Abb. 3

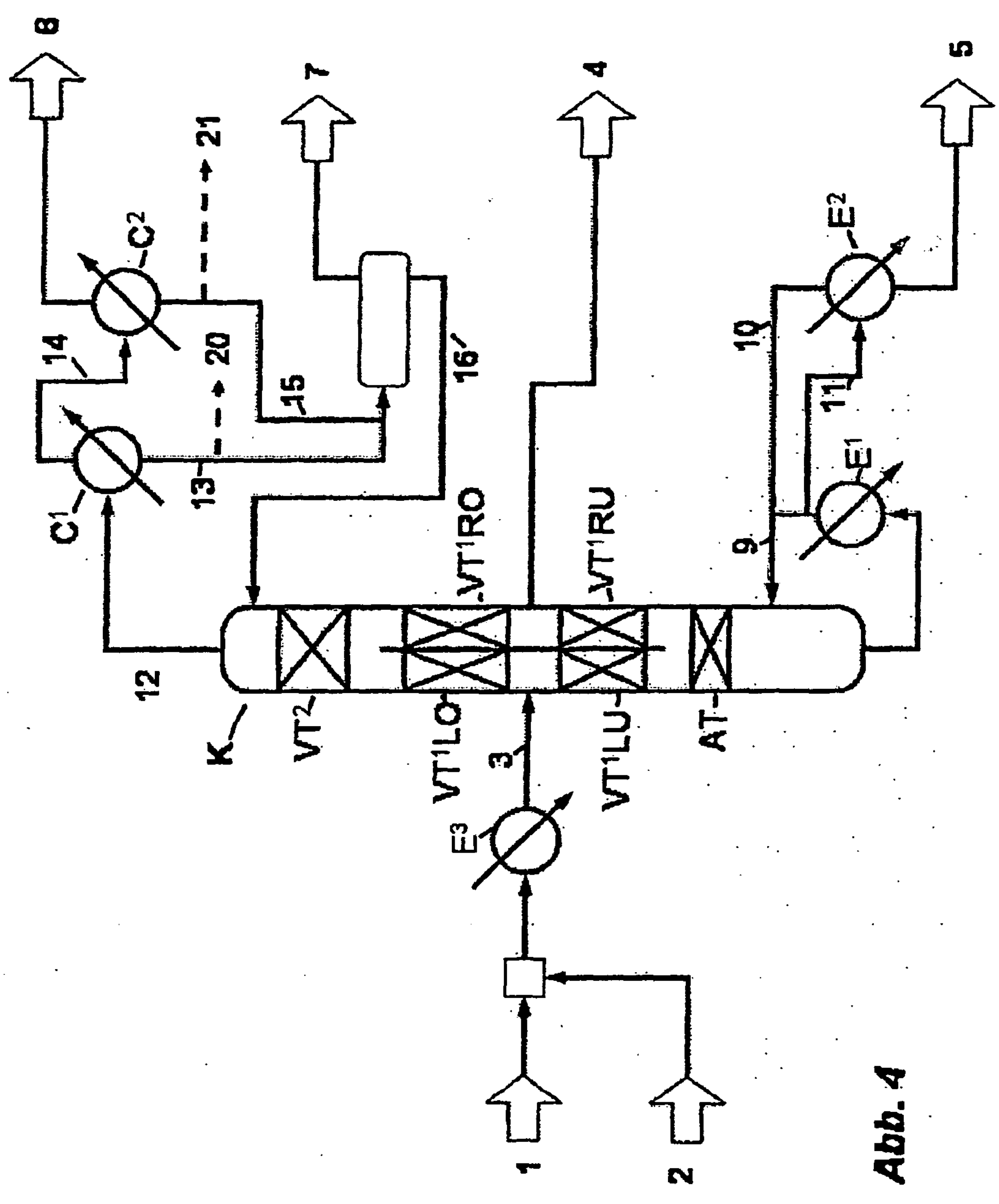

1
2
E3
3
VT1LO
VT1LU
K
VT2
AT
12
C1
13
20
14
C2
21
8
15
7
16'
4
VT1RO
VT1RU
9
E1
11
10
E2
5


Abb. 4

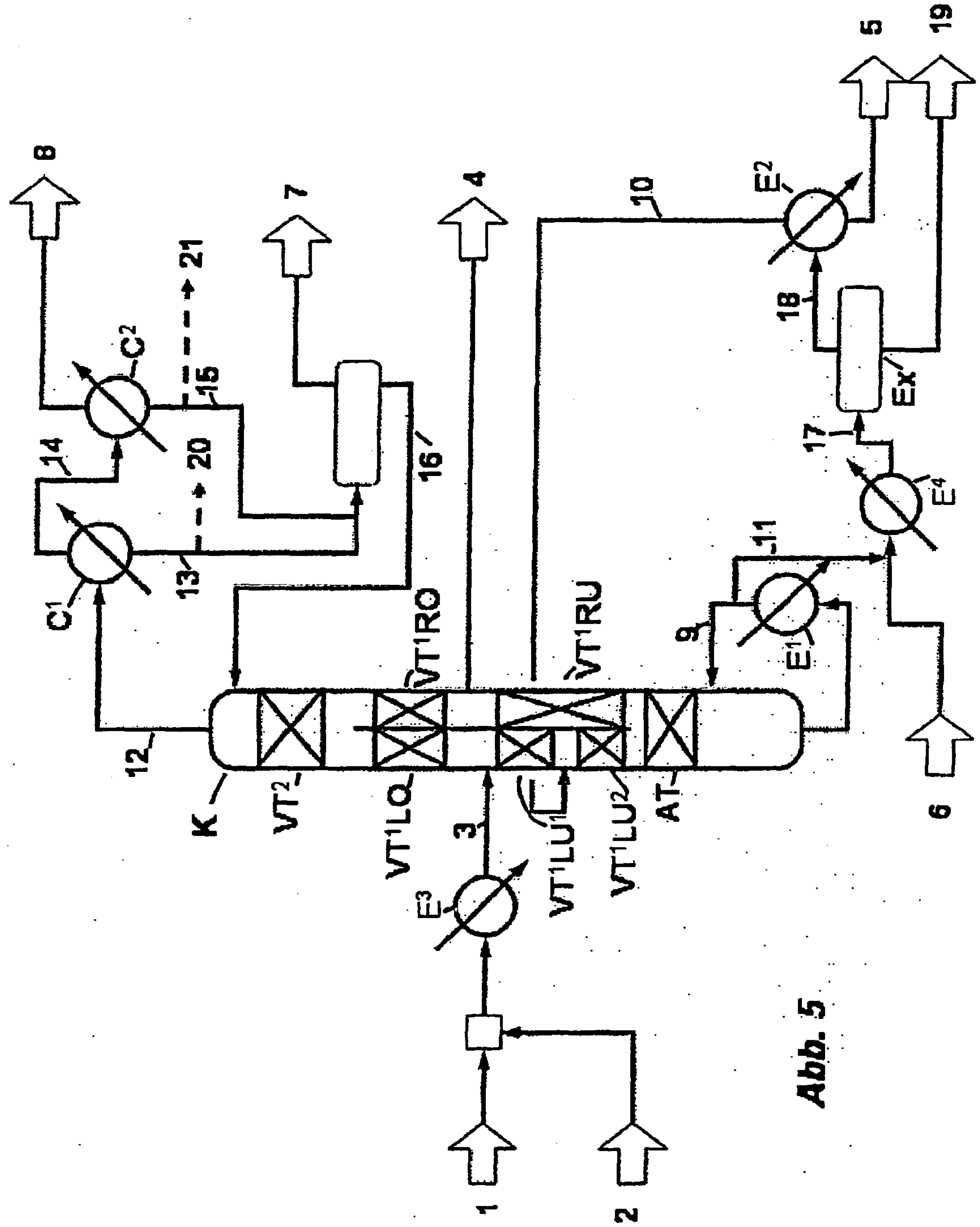
1
2
3
4
5
6
7
8
9
10
11
12
13
14
15
16
17
18
19
20
21
K
$VT^2$
$VT^1LO$
$VT^1LU^1$
$VT^1LU^2$
$VT^1RO$
$VT^1RU$
AT
$C^1$
$C^2$
$E^1$
$E^2$
$E^3$
$E^4$
Ex

*Abb. 5*

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2201528 A
- DE 3414714 A
- US 3136818 A
- EP 0696573 B1
- EP 0696574 B1
- JP 49035341 A
- JP 8295654 A
- US 2005080294 A
- JP 2005350388 A
- EP 07075103 A
- EP 07075104 A


### In der Beschreibung aufgeführte Nicht-Patentliteratur


- **G. KAIBEL.** Distillation Columns with Vertical Partitions. *Chem. Eng. Technol.,* 1987, vol. 10, 92-98
- **G. KAIBEL.** Industrieller Einsatz von Trennwandkolonnen und thermisch gekoppelten Destillationskolonnen. *Chemie Ingenieur Technik,* 2003, vol. 75, 1165-1166